(19) Europäisches Patentamt
European Patent Office
Office européen des brevets

(11) Veröffentlichungsnummer: **0 182 056**
**B1**

(12) # EUROPÄISCHE PATENTSCHRIFT

(45) Veröffentlichungstag der Patentschrift: **04.01.89**

(21) Anmeldenummer: **85112564.1**

(22) Anmeldetag: **04.10.85**

(51) Int. Cl.⁴: **C 07 C 69/54**, C 07 C 69/52, C 07 C 67/08, C 07 C 67/343, C 09 K 15/08

(54) **UV-Licht-Stabilisator auf Benzophenon-Basis.**

(30) Priorität: **18.10.84 DE 3438190**

(43) Veröffentlichungstag der Anmeldung: **28.05.86 Patentblatt 86/22**

(45) Bekanntmachung des Hinweises auf die Patenterteilung: **04.01.89 Patentblatt 89/1**

(84) Benannte Vertragsstaaten: **CH DE GB IT LI**

(56) Entgegenhaltungen:
**DE-A-1 569 595**
**FR-A-1 411 903**

**CHEMICAL ABSTRACTS, Band 92, Nr. 16, 21 April 1980, Seite 34, Zusammenfassung Nr. 129819c, Columbus, Ohio, US; V.P.BIRYUKOV et al.: "Effectiveness of new light stabilizers for polymeric materials", & KOZH.-OBUVN. PROM-ST. 1979, 21(12), 31-3**

(73) Patentinhaber: **RIEDEL- DE HAEN AKTIENGESELLSCHAFT, Wunstorfer Strasse 40, D-3016 Seelze 1 (DE)**

(72) Erfinder: **Eiglmeier, Kurt, Dr., Kattowitzer Weg 5, D-3057 Neustadt (DE)**
Erfinder: **Schulz, Joachim, Königsberger Strasse 7, D-3255 Pohle (DE)**

(74) Vertreter: **Urbach, Hans- Georg, Dr., p.A. CASSELLA AKTIENGESELLSCHAFT Postfach, D-6000 Frankfurt am Main 61 (DE)**

EP 0 182 056 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft Ester aus 1,3-Bis(4-benzoyl-3-hydroxyphenoxy)-2-propanol und einer ungesättigten Carbonsäure der Formel 2

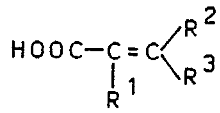

(2)

in der

$R^1$ ein Wasserstoffatom, einen Phenylrest, einen Alkylrest mit 1 bis 12 Kohlenstoffatomen oder eine Cyangruppe bedeutet und

$R^2$ und $R^3$ jeweils für sich ein Wasserstoffatom, einen Alkylrest mit 1 bis 8 Kohlenstoffatomen oder einen gegebenenfalls mit einem niederen Alkyl- oder Alkoxyrest substituierten Phenylrest oder gemeinsam einen Alkylenrest mit 4 oder 5 Kohlenstoffatomen bedeuten.

Die Erfindung betrifft auch ein Verfahren zur Herstellung der erfindungsgemäßen Ester. Die Erfindung betrifft ferner einen Stabilisator gegen den Einfluß von UV-Licht, der dadurch gekennzeichnet ist, daß er als Wirkstoff einen erfindungsgemäßen Ester enthält. Weiterhin betrifft die Erfindung die Verwendung eines erfindungsgemäßen Esters zum Stabilisieren eines Kunststoffs gegen den Einfluß von UV-Licht und die Verwendung eines erfindungsgemäßen Esters als Monomer zur Herstellung von Homopolymeren und Copolymeren, sowie ein Polymer, bestehend aus oder enthaltend wiederkehrende Einheiten, die von einem erfindungsgemäßen Ester abgeleitet sind.

Es ist bekannt, daß 1,3-Bis(4-benzoyl-3-hydroxyphenoxy)-2-propanol als Fotostabilisator für Polymere dient (vgl. Chem. Abstracts 86 (1977), 106170 f). Die Wirksamkeit dieses Stabilisators läßt jedoch noch zu wünschen übrig (vgl. Chem. Abstracts 92 (1980), 129819 c). Ferner ist bekannt, daß Glycidyl(meth)acrylat mit einem Polyhydroxybenzophenon ein Addukt bildet, das mit Ethylacrylat und Methylmethacrylat copolymerisiert werden kann (vgl. Chem. Abstracts 98 (1983), 127362 y). Dieses Copolymer wird zur Beschichtung von Polyester-Folien eingesetzt.

Aufgabe der Erfindung ist die Bereitstellung von Verbindungen, die mindestens zwei Benzophenongruppen und eine Doppelbindung im Molekül aufweisen und UV-Licht absorbieren.

Diese Aufgabe wird durch die Bereitstellung der erfindungsgemäßen Ester gelöst, welche die Struktur der Formel (1)

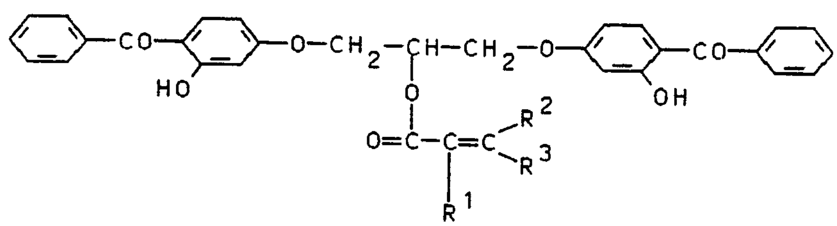

(1)

besitzen.

Bevorzugte erfindungsgemäße Ester sind solche, bei denen $R^2$ und/oder $R^3$ einen durch einen Alkyl- oder Alkoxyrest mit 1 bis 4 C-Atomen substituierten Phenylrest bedeuten.

Das erfindungsgemäße Verfahren zur Herstellung der erfindungsgemäßen Ester ist dadurch gekennzeichnet, daß bei einer Temperatur von 0 bis 150° C

a) 1,3-Bis(4-benzoyl-3-hydroxyphenoxy)-2-propanol in einem inerten organischen Lösemittel in Gegenwart eines sauren Katalysators mit einer ungesättigten Carbonsäure der Formel 2 umgesetzt wird oder
b) 1,3-Bis(4-benzoyl-3-hydroxyphenoxy)-2-propanol gegebenenfalls in einem inerten organischen Lösemittel in Gegenwart eines sauren Katalysators zunächst mit einer gesättigten Carbonsäure, die eine aktive Methylengruppe aufweist, umgesetzt wird und der resultierende gesättigte Ester dann in Gegenwart eines Katalysators mit einer Ketoverbindung kondensiert wird.

Die Herstellung der erfindungsgemäßen Ester erfolgt in einem einstufigen (Variante a) oder in einem zweistufigen Verfahren (Variante b); die Umsetzungen mit der jeweiligen Säure werden in einem inerten organischen Lösemittel in Gegenwart eines sauren Katalysators bei einer Temperatur von 0 bis 150° C, vorzugsweise 50 bis 120° C, durchgeführt.

Das einstufige Verfahren besteht aus einer Veresterung von 1,3-Bis(4-benzoyl-3-hydroxyphenoxy)-2-propanol mit einer ungesättigten Carbonsäure der Formel (2), die in einem Überschuß bis zu 10 Mol pro Mol 1,3-Bis(4-benzoyl-3-hydroxyphenoxy)-2-propanol, vorzugsweise in einer Menge von 3 bis 8 Mol pro Mol 1,3-Bis(4-benzoyl-3-hydroxyphenoxy)-2-propanol eingesetzt wird.

Verbindungen der Formel (2) sind insbesondere Acrylsäure und substituierte Acrylsäuren, z. B.

Methacrylsäure, Crotonsäure, 2,2-Diphenylacrylsäure, 2,2-Dimethacrylsäure und 1-Cyanacrylsäure.

Vorzugsweise wird bei dem Verfahren a) als ungesättigte Carbonsäure der Formel (2) eine solche verwendet, bei der $R^2$ und/oder $R^3$ einen durch einen Alkyl- oder Alkoxyrest mit 1 bis 4 C-Atomen substituierten Phenylrest bedeuten.

Das zweistufige Verfahren besteht aus einer Veresterung von 1,3-Bis(4-benzoyl-3-hydroxyphenoxy)-2-propanol mit einer gesättigten Carbonsäure, vorzugsweise Monocarbonsäure, die eine aktive Methylengruppe aufweist, und einer daran anschließenden Kondensation des resultierenden gesättigten Esters mit einer Ketoverbindung. Als gesättigte Carbonsäure wird hierbei insbesondere Cyanessigsäure verwendet. Geeignete Ketoverbindungen sind symmetrische oder asymmetrische aliphatische Ketone, cyclische aliphatische Ketone, aromatische Ketone und aliphatisch-aromatische Ketone sowie aliphatische und aromatische Aldehyde. Beispiele hierfür sind Aceton, Benzophenon, Cyclohexanon, Methylethylketon, Formaldehyd und Benzaldehyd.

Die Carbonsäure und die Ketoverbindung werden jeweils in einem molaren Überschuß eingesetzt, und zwar bis zu 10 Mol pro Mol Reaktionskomponente, vorzugsweise in einer Menge von 3 bis 8 Mol pro Mol Reaktionskomponente.

Das inerte organische Lösemittel ist ein aromatischer Kohlenwasserstoff mit 6 bis 8 Kohlenstoffatomen, der mit einem Halogenatom, vorzugsweise Chloratom, substituiert sein kann, oder ein aliphatischer Halogenkohlenwasserstoff, vorzugsweise Chlorkohlenwasserstoff mit 1 bis 2 Kohlenstoffatomen. Geeignete Lösemittel sind beispielsweise Benzol, Toluol, Xylol, Chlorbenzol, Chloroform, Tetrachlormethan und 1,2-Dichlorethan. Das Lösemittel wird in einer Menge von 1 bis 10 Liter pro Mol des substituierten Propanols verwendet; eine Menge von 5 bis 8 Liter/Mol ist bevorzugt. Bei der Durchführung des zweistufigen Verfahrens ist der Einsatz eines inerten organischen Lösemittels nicht erforderlich, wenn eine flüssige Ketoverbindung verwendet wird.

Als Katalysator für die Veresterungsreaktion dient ein saurer Katalysator, nämlich eine anorganische Säure oder eine organische Säure oder insbesondere eine organische Sulfonsäure, die Halogenatome, vorzugsweise Fluoratome, aufweisen kann; ein saurer Ionenaustauscher ist ebenfalls geeignet. Geeignete Katalysatoren sind beispielsweise p-Toluolsulfonsäure und vor allem Trifluormethansulfonsäure. Der Katalysator wird in einer Menge von 1 bis 10, vorzugsweise 3 bis 6 Gewichtsprozent eingesetzt (bezogen auf das substituierte Propanol). Für die Kondensationsreaktion ist der gleiche Katalysator anwendbar. Es eignet sich aber auch ein basischer Katalysator, nämlich ein Alkaliacetat wie Natriumacetat und Kaliumacetat sowie Ammoniumacetat. Der basische Katalysator wird gegebenenfalls in einer Menge von 5 bis 20, vorzugsweise 10 bis 15 Gewichtsprozent eingesetzt (bezogen auf den gesättigten Ester).

Die erfindungsgemäßen Verbindungen sind kristalline Substanzen, die mit guter Ausbeute und in hoher Reinheit erhalten werden. (Der Reinheitsgrad wird durch Hochdruckflüssigkeitschromatographie - HPLC - ermittelt.) Sie eignen sich als Stabilisatoren zum Schutz von Kunststoffen, beispielsweise Polyacrylsäureester, Polymethacrylsäureester, Polystyrol und ABS-Polymere, gegen die Einwirkung von Ultraviolett-Licht. Bei der Herstellung von Kunststoffteilen durch thermoplastische Formgebung können die Stabilisatoren mit der plastischen Masse vermischt werden, so daß ein Lichtschutz in der Masse und nicht nur an der Oberfläche des Gegenstandes erzielt wird. Die Verbindungen sind durch geringen Dampfdruck und geringe Migrationsneigung ausgezeichnet.

Die erfindungsgemäßen Verbindungen sind polymerisierbar und eignen sich als Monomere zur Herstellung von Homopolymeren und Copolymeren, insbesondere bei der Polymerisation von einzelnen Vinylverbindungen oder Gemischen von Vinylverbindungen, z. B. Acrylsäureester, Methacrylsäureester, Acrylnitril, Styrol und α-Methylstyrol. Bei der Homopolymerisation bilden sie je nach den Reaktionsbedingungen weiche bis glasartige Polymere, die beispielsweise als Beschichtungsmittel geeignet sind.

Die Erfindung wird durch die folgenden Beispiele näher erläutert. Prozentangaben beziehen sich jeweils auf das Gewicht. UV-Spektren wurden jeweils mit einem handelsüblichen Spektrophotometer gemessen. Dabei betrug die Konzentration der zu untersuchenden Substanz 0,001 Gewichtsprozent, als Lösemittel wurde Dimethylformamid verwendet, und die Messung wurde bei einer Schichttiefe von 1 cm durchgeführt.

**Beispiel 1**

Eine Lösung von 48,5 g (0,1 mol) 1,3-Bis(4-benzoyl-3-hydroxyphenoxy)-2-propanol und 43,6 g (0,6 mol) Acrylsäure in 600 ml Toluol wurde nach Zusatz von 3 g Trifluormethansulfonsäure, 0,1 g Hydrochinon und 0,1 g Hydrochinonmonomethylether 3 Stunden lang auf eine Temperatur von 105 bis 110°C erhitzt. Nach dem Erkalten auf Raumtemperatur wurde das Reaktionsgemisch mit 1,5 l Wasser gewaschen. Anschließend wurde das Toluol bei einem Druck von 20 mbar aus dem Reaktionsgemisch abdestilliert. Der verbleibende ölige Rückstand wurde in heißem Ethanol gelöst. Nach dem Erkalten dieser Lösung auf Raumtemperatur erhielt man 40 g (75 Prozent der Theorie) [1,3-Bis(4-benzoyl-3-hydroxyphenoxy)-2-propanyl]-acrylsäureester in Form farbloser Kristalle mit einem Schmelzpunkt von 98 bis 100°C und einer Reinheit von 99 Prozent (HPLC). Das UV-Spektrum ergab zwei Absorptionsmaxima bei Wellenlängen von 287 nm (Extinktion 0,520; Extinktionskoeffizient 28100) und 324 nm (Extinktion 0,335; Extinktionskoeffizient 18100).

**Beispiel 2**

Analog Beispiel 1 wurden 97 g (0,2 mol) 1,3-Bis(4-benzoyl-3-hydroxyphenoxy)-2-propanol mit 103 g (1,2 mol) Methacrylsäure verestert. Man erhielt nach der Kristallisation aus Ethanol 86 g (78 Prozent der Theorie) [1,3-Bis(4-benzoyl-3-hydroxyphenoxy)-2-propanyl]-methacrylsäureester in Form farbloser Kristalle mit einem Schmelzpunkt von 138 bis 139°C und einer Reinheit von 99 Prozent (HPLC). Das UV-Spektrum ergab zwei Absorptionsmaxima bei Wellenlängen von 287 nm (Extinktion 0,517; Extinktionskoeffizient 28570) und 324 nm (Extinktion 0,333; Extinktionskoeffizient 18400).

**Beispiel 3**

a) Eine Lösung von 48,5 g (0,1 mol) 1,3-Bis(4-benzoyl-3-hydroxyphenoxy)-2-propanol und 51 g (0,6 mol) Cyanessigsäure in 750 ml 1,2-Dichlorethan wurde nach Zusatz von 3 g Trifluormethansulfonsäure 9 Stunden lang auf eine Temperatur von 80 bis 85°C erhitzt. Nach dem Erkalten auf Raumtemperatur wurde das Reaktionsgemisch dreimal mit je 400 ml Wasser gewaschen. Anschließend wurde das 1,2-Dichlorethan bei einem Druck von 50 mbar und einer Temperatur von 35 bis 45°C aus dem Reaktionsgemisch abdestilliert. Der verbleibende Rückstand wurde aus Acetonitril umkristallisiert. Nach dem Trocknen erhielt man 48 g (87 Prozent der Theorie) [1,3-Bis(4-benzoyl-3-hydroxyphenoxy)-2-propanyl]-cyanessigsäureester in Form schwach gelb gefärbter, feiner Kristalle mit einem Schmelzpunkt von 167 bis 168°C und einer Reinheit von 99 Prozent (HPLC).

b) Eine Lösung von 55,2 g (0,1 mol) [1,3-Bis(4-benzoyl-3-hydroxyphenoxy)-2-propanyl]-cyanessigsäureester in 300 ml Aceton wurde nach Zusatz von 1 g Trifluormethansulfon-säure 3 Stunden lang auf eine Temperatur von 56°C erhitzt. Nach dem Erkalten auf Raumtemperatur wurde der erhaltene kristalline Niederschlag abgesaugt und aus Aceton umkristallisiert. Nach dem Trocknen erhielt man 51 g (86 Prozent der Theorie) [1,3-Bis-(4-benzoyl-3-hydroxyphenoxy)-2-propanyl]-2-cyano-3,3-dimethylacrylsäureester in Form farbloser Kristalle mit einem Schmelzpunkt von 145 bis 147°C und einer Reinheit von 99 Prozent (HPLC). UV-, IR- und NMR-Spektren bestätigten die Struktur der erhaltenen Verbindung.

**Beispiel 4**

Analog Beispiel 1 wurden 97 g (0,2 mol) 1,3-Bis(4-benzoyl-3-hydroxyphenoxy)-2-propanol mit 120 g (1,2 mol) 2,2-Dimethylacrylsäure verestert. Man erhielt nach der Kristallisation aus Aceton 86 g (76 Prozent der Theorie) [1,3-Bis(4-benzoyl-3-hydroxyphenoxy)-2-propanyl]-3,3-dimethylacrylsäureester in Form schwach gelb gefärbter feiner Kristalle mit einem Schmelzpunkt von 148 bis 150°C und einer Reinheit von 99 Prozent (HPLC). UV- , IR- und NMR-Spektren bestätigten die Struktur der erhaltenen Verbindung.

**Beispiel 5**

Analog Beispiel 1 wurden 97 g (0,2 Mol) 1,3-Bis(4-benzoyl-3-hydroxyphenoxy)-2-propanol mit 103 g (1,2 mol) Crotonsäure verestert. Man erhielt nach der Kristallisation aus einem Aceton/Ethanol-Gemisch (Volumverhältnis 1 : 2) 83 g (75 Prozent der Theorie) [1,3-Bis(4-benzoyl-3-hydroxyphenoxy)-2-propanyl]-crotonsäureester in Form feiner weißer Kristalle mit einem Schmelzpunkt von 99 bis 103°C und einer Reinheit von 99 Prozent (HPLC). UV-, IR- und NMR-Spektren bestätigten die Struktur der erhaltenen Verbindung.

**Beispiel 6**

Eine Lösung von 110 g (0,2 mol) [1,3-Bis(4-benzoyl-3-hydroxyphenoxy)-2-propanyl]-methacrylsäureester in 1,2 l Aceton, die 1,5 g 50-prozentiges Benzoylperoxid (wäßrig) enthielt, wurde unter einer Stickstoff-atmosphäre 16 Stunden lang unter Rückfluß erhitzt. Nach Abkühlen des Reaktionsgemisches auf Raumtemperatur wurde das feste Reaktionsprodukt abgetrennt, zweimal mit je 500 ml Aceton gewaschen und im Umlufttrockenschrank bei einer Temperatur von 60°C getrocknet. Das getrocknete Produkt war eine feste, glasartige Masse, die nach dem Mahlen zu einem schwach gelb gefärbten Pulver eine Glasumwandlungstemperatur von 107°C zeigte. Die Ausbeute betrug 105,9 g (96 Prozent der Theorie). Der Rest-Monomer-Gehalt betrug weniger als 0,01 Prozent. Das UV-Spektrum ergab zwei Absorptionsmaxima bei Wellenlängen von 287 nm (Extinktion 0,523) und 324 nm (Extinktion 0,330).

**Beispiel 7**

Analog Beispiel 6 wurden 107,7 g (0,2 mol) [1,3-Bis(4-benzoyl-3-hydroxyphenoxy)-2-propanyl]-acrylsäurreester in 0,8 l Aceton behandelt. Nach entsprechender Aufarbeitung des Reaktionsproduktes erhielt man 97,3 g (91 Prozent der Theorie) eines schwach gelb gefärbten Pulvers mit einer Glasumwandlungstemperatur von 88° C. Der Rest-Monomer-Gehalt betrug weniger als 0,01 Prozent. Das UV-Spektrum ergab zwei Absorptionsmaxima bei Wellenlängen von 287 nm (Extinktion 0,523) und 324 nm (Extinktion 0,330).

**Beispiel 8**

Ein Gemisch aus 0,6 g [1,3-Bis(4-benzoyl-3-hydroxyphenoxy)-2-propanyl]-methacrylsäureester und 20 g Methacrylsäuremethylester wurde nach Zugabe von 0,5 g 50-prozentigen Benzoylperoxids (wäßrig) unter einer Stickstoffatmosphäre 8 Stunden lang in einer Glaskammer auf eine Temperatur von 70° C erhitzt. Man erhielt einen festen transparenten Polymerisatblock. Das IR-Spektrum zeigte bei einer Wellenzahl von 1620 $cm^{-1}$ eine für [1,3-Bis(4-benzoyl-3-hydroxyphenoxy)-2-propanyl]-methacrylsäurreester charakteristische Bande. Aussehen, Transparenz, Festigkeit und chemische Beständigkeit entsprachen Polymethacrylat.

**Anwendungsbeispiel**

Eine handelsübliche Farbkopie (Format 18 cm x 24 cm), die einen Graukeil und eine Farbabstufung aufwies, wurde mit einem handelsübichen Acrylatlack überzogen, der [1,3-Bis-(4-benzoyl-3-hydroxyphenoxy)-2-propanyl]-methacrylsäureester gemäß Beispiel 2 enthielt (Probe a). Die so behandelte Farbkopie wurde 2000 Stunden lang mit einer handelsüblichen UV-Licht-Lampe bestrahlt, wobei der Abstand 50 cm betrug.

Zum Vergleich wurden eine unbehandelte Farbkopie (Probe b) und eine mit einem handelsüblichen Acrylatlack behandelte Farbkopie (Probe c) der gleichen Bestrahlung unterworfen. Als Bezug herangezogen wurde eine im Dunkeln gelagerte, unbehandelte Farbkopie (Probe d).

Die Auswertung nach der Bestrahlung ergab folgende Befunde:

| | |
|---|---|
| Probe a: | Keine Farbausbleichung, kein Vergrauen der Weißtöne; |
| Probe b: | Sehr starke Farbausbleichung, sehr starkes Vergrauen der Weißtöne; |
| Probe c: | Sehr starke Farbausbleichung, sehr starkes Vergrauen der Weißtöne; |
| Probe d: | Keine Farbausbleichung, kein Vergrauen der Weißtöne. |

**Patentansprüche**

1. Ester aus 1,3-Bis(4-benzoyl-3-hydroxyphenoxy)-2-propanol und einer ungesättigten Carbonsäure der Formel 2

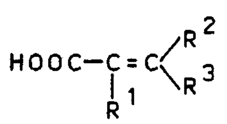

(2)

in der

$R^1$ ein Wasserstoffatom, einen Phenylrest, einen Alkylrest mit 1 bis 12 Kohlenstoffatomen oder eine Cyangruppe bedeutet und

$R^2$ und $R^3$ jeweils für sich ein Wasserstoffatom, einen Alkylrest mit 1 bis 8 Kohlenstoffatomen oder einen gegebenenfalls mit einem niederen Alkyl- oder Alkoxyrest substituierten Phenylrest oder gemeinsam einen Alkylenrest mit 4 oder 5 Kohlenstoffatomen bedeuten.

2. Ester nach Anspruch 1, dadurch gekennzeichnet, daß $R^2$ und/oder $R^3$ einen durch einen Alkyl- oder Alkoxyrest mit 1 bis 4 C-Atomen substituierten Phenylrest bedeuten.

3. Verfahren zur Herstellung eines Esters aus 1,3-Bis(4-benzoyl-3-hydroxyphenoxy)-2-propanol und einer ungesättigten Carbonsäure der Formel 2

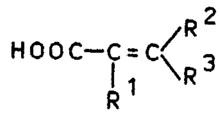

(2)

in der

$R^1$ ein Wasserstoffatom, einen Phenylrest, einen Alkylrest mit 1 bis 12 Kohlenstoffatomen oder eine Cyangruppe bedeutet und

$R^2$ und $R^3$ jeweils für sich ein Wasserstoffatom, einen Alkylrest mit 1 bis 8 Kohlenstoffatomen oder einen gegebenenfalls mit einem niederen Alkyl- oder Alkoxyrest substituierten Phenylrest oder gemeinsam einen Alkylenrest mit 4 oder 5 Kohlenstoffatomen bedeuten, dadurch gekennzeichnet, daß bei einer Temperatur von 0 bis 150°C

a) 1,3-Bis(4-benzoyl-3-hydroxyphenoxy)-2-propanol in einem inerten organischen Lösemittel in Gegenwart eines sauren Katalysators mit einer ungesättigten Carbonsäure der Formel 2 umgesetzt wird oder

b) 1,3-Bis(4-benzoyl-3-hydroxyphenoxy)-2-propanol gegebenenfalls in einem inerten organischen Lösemittel in Gegenwart eines sauren Katalysators zunächst mit einer gesättigten Carbonsäure, die eine aktive Methylengruppe aufweist, umgesetzt wird und der resultierende gesättigte Ester dann in Gegenwart eines Katalysators mit einer Ketoverbindung kondensiert wird.

4. Verfahren nach Anspruch 3, dadurch gekennzeichnet, daß eine Carbonsäure der Formel 2 verwendet wird, wobei $R^2$ und/oder $R^3$ einen durch einen Alkyl- oder Alkoxyrest mit 1 bis 4 C-Atomen substituierten Phenylrest bedeuten.

5. Verfahren nach Anspruch 4, dadurch gekennzeichnet, daß als gesättigte Carbonsäure Cyanessigsäure verwendet wird.

6. Stabilisator gegen den Einfluß von UV-Licht, dadurch gekennzeichnet, daß er als Wirkstoff einen Ester gemäß Anspruch 1 enthält.

7. Verwendung eines Esters gemäß Anspruch 1 zum Stabilisieren eines Kunststoffes gegen den Einfluß von UV-Licht.

8. Verwendung eines Esters gemäß Anspruch 1 als Monomer zur Herstellung von Homopolymeren und Copolymeren.

9. Polymer, bestehend aus oder enthaltend wiederkehrende Einheiten, die von einem Ester gemäß Anspruch 1 abgeleitet sind.

**Claims**

1. An ester formed from 1,3-bis-(4-benzoyl-3-hydroxy-phenoxy)-2-propanol and an unsaturated carboxylic acid of the formula 2

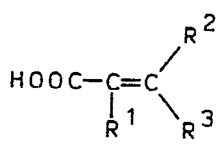

(2)

in which

$R^1$ denotes a hydrogen atom, a phenyl radical, an alkyl radical having 1 to 12 carbon atoms or a cyano group and

$R^2$ and $R^3$ independently of one another denote a hydrogen atom, an alkyl radical having 1 to 8 carbon atoms or a phenyl radical which may be substituted by a lower alkyl or alkoxy radical, or together denote an alkylene radical having 4 or 5 carbon atoms.

2. The ester of Claim 1, characterised in that $R^2$ and $R^3$ denote a phenyl radical substituted by an alkyl or alkoxy radical having 1 to 4 C atoms.

3. Process for preparing an ester formed from 1,3-bis-(4-benzoyl-3-hydroxy-phenoxy)-2-propanol and an unsaturated carboxylic acid of the formula 2

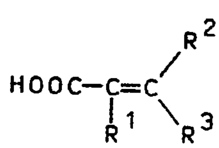

(2)

in which

$R^1$ denotes a hydrogen atom, a phenyl radical, an alkyl radical having 1 to 12 carbon atoms or a cyano group and

$R^2$ and $R^3$ independently of one another denote a hydrogen atom, an alkyl radical having 1 to 8 carbon atoms

or a phenyl radical which may be substituted by a lower alkyl or alkoxy radical, or together denote an alkylene radical having 4 or 5 carbon atoms, which comprises reacting at a temperature of 0 to 150°C

a) 1,3-bis-(4-benzoyl-3-hydroxy-phenoxy)-2-propanol with an unsaturated carboxylic acid of formula 2 in an inert organic solvent, in the presence of an acid catalyst, at a temperature of 0 to 150°C, or

b) first reacting, at a temperature of 0 to 150°C, 1,3-bis-(4-benzoyl-3-hydroxy-phenoxy)-2-propanol, optionally in an inert organic solvent, in the presence of an acid catalyst, with a saturated carboxylic acid containing an active methylene group, and then subjecting the resulting saturated ester to a condensation reaction with a keto compound in the presence of a catalyst.

4. The process of Claim 3, characterised in that a carboxylic acid of formula 2 is used, wherein $R^2$ and/or $R^3$ denote a phenyl radical which is substituted by an alkyl or alkoxy radical having 1 to 4 C atoms.

5. The process as claimed in Claim 4, wherein the saturated carboxylic acid used is cyanoacetic acid.

6. A stabilizer against the effects of UV light, characterised in that it contains, as the active compound, an ester as claimed in Claim 1.

7. Use of an ester according to Claim 1 for stabilizing a plastic against the action of UV light.

8. Use of an ester according to Claim 1 as a monomer for producing homopolymers and copolymers.

9. A polymer composed of or containing recurring units derived from an ester according to Claim 1.

## Revendications

1. Esters dérivant du bis-(benzoyl-4 hydroxy-3 phénoxy)-1,3 propanol-2 et d'un acide carboxylique insaturé répondant à la formule 2

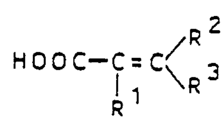

(2)

dans laquelle

$R^1$ represente un atome d'hydrogène, un radical phényle, un radical alkyle contenant de 1 à 12 atomes de carbone ou un radical cyano, et

$R^2$ et $R^3$ représentent chacun un atome d'hydrogène, un radical alkyle contenant de 1 à 8 atomes de carbone ou un radical phényle éventuellement porteur d'un radical alkyle ou alcoxy inférieur, ou encore forment ensemble un radical alkylène contenant 4 ou 5 atomes de carbone.

2. Esters selon la revendication 1 caractérisés en ce que $R^2$ et/ou $R^3$ représente(nt) un radical phényle qui porte un radical alkyle ou alcoxy contenant de 1 à 4 atomes de carbone.

3. Procédé pour préparer un ester dérivant du bis-(benzoyl-4 hydroxy-3 phénoxy)-1,3 propanol-2 et d'un acide carboxylique insaturé répondant à la formule 2:

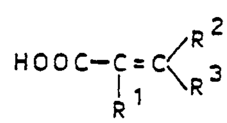

(2)

dans laquelle

$R^1$ représente un atome d'hydrogène, un radical phényle, un radical alkyle contenant de 1 à 12 atomes de carbone ou un radical cyano, et

$R^2$ et $R^3$ représentent chacun un atome d'hydrogène, un radical alkyle contenant de 1 à 8 atomes de carbone ou un radical phényle éventuellement porteur d'un radical alkyle ou alcoxy inférieur, ou encore forment ensemble un radical alkylène contenant 4 ou 5 atomes de carbone,

procédé caractérisé en ce qu'on fait réagir à une température de 0 à 150°C:

a) le bis-(benzoyl-4 hydroxy-3 phénoxy)-1,3 propanol-2, dans un solvant organique inerte, en présence d'un catalyseur acide, avec un acide carboxylique insaturé de formule 2, ou

b) le bis-(benzoyl-4 hydroxy-3 phénoxy)-1,3 propanol-2, éventuellement dans un solvant organique inerte, en présence d'un catalyseur acide d'abord avec un acide carboxylique saturé contenant un radical méthylène actif, puis on condense l'ester saturé formé, en présence d'un catalyseur avec un composé cétonique.

4. Procédé selon la revendication 3 caractérisé en ce qu on utilise un acide carboxylique de formule 2 dans lequel $R^2$ et/ou $R^3$ représente(nt) un radical phényle qui porte un radical alkyle ou alcoxy contenant de 1 à 4 atomes de carbone.

5. Procédé selon la revendication 4 caractérisé en ce qu'on utilise l'acide cyanacétique comme acide carboxylique saturé.

6. Stabilisant contre l'action de la lumière ultraviolette, stabilisant caractérisé en ce qu'il contient, comme matière active, un ester selon la revendication 1.

7. Application d'un ester selon la revendication 1 pour la stabilisation d'une matière plastique contre l'action

de la lumière ultraviolette.

8. Application d'un ester selon la revendication 1 comme monomère pour la préparation d'homopolymères et de copolymères.

9. Polymère contenant ou constitué de motifs répétés qui dérivent d'un ester selon la revendication 1.